# EUROPEAN PATENT SPECIFICATION

(11) **EP 0 334 046 B1**
(45) Date of publication and mention of the grant of the patent: **18.06.1997**
(21) Application number: 89103225.2
(22) Date of filing: 24.02.1989
(51) Int. Cl.: A61L 27/00, A61L 31/00

(54) **Surgical composite structure having absorbable and nonabsorbable components**
Chirurgische zusammengesetzte Strukturen, die absorbierbare und nichtabsorbierbare Bestandteile enthalten
Structure chirurgicale composite contenant des composants absorbables et non absorbables

(30) Priority: 24.03.1988 US 172607
(43) Date of publication of application: 27.09.1989
(73) Proprietor: AMERICAN CYANAMID COMPANY, Wayne, NJ 07470-8426 (US)
(72) Inventor: Devereux, Dennis F., Pennington New Jersey 08534 (US); Landi, Henry P., Yorktown Heights New York 10598 (US)
(74) Representative: Wächtershäuser, Günter, Prof. Dr.

(56) References cited:
- EP-A- 0 050 215
- EP-A- 0 159 502
- WO-A-86/00533
- GB-A- 1 008 193

## Description

This invention relates to a surgical composite structure for mammalian tissue. GB-A-l,008,l93 teaches nonabsorbable fabrics used as surgical implants which are coated with absorbable collagen fibrils for facilitating ingrowth of tissue. WO 86/00533 discloses surgical composite structures comprising a non-absorbable material made of a fabric of fibers which is incorporated in a matrix of biodegradable material. The structure of the invention is manufactured from two or more biocompatible polymers. At least one of the polymers in the structure is nonabsorbable.

The nonabsorbable portion of the composite structure acts as a reinforcement material. Ingrowth of natural tissue is enhanced by the controlled degradation of the absorbable portion.

The surgical composite structure of this invention is useful in the repair of defects to the abdominal wall of a mammal. The surgical composite structure of this invention may be useful in preventing hernia formation; and specifically in preventing hernia formation in an infected area.

A surgical composite structure for mammalian tissue has been invented in accordance with claim l. The composite structure comprises:
a) a nonabsorbable reinforcing component prepared from one or more fibers, at least one of the fibers manufactured from a polymer selected from the group consisting of a fiber forming polyetherester and blends of the same, and
b) a synthetic bioabsorbable component comprising a random copolymer prepared from at least the monomers glycolide and l,3-dioxane 2-one.

Furthermore, the nonabsorbable component may also comprise members selected from the group consisting of a fiber forming fluoropolymer, a polybutester, a polyester, a polyamide and a polydefin

Furthermore, the bioabsorbable component may also comprise a polymer prepared from one or more monomers selected from the group consisting of lactides, carbonates and lactones. The lactides may be 3,6-dimethyl-2,5-p-dioxanedione; and the lactones may be ε-caprolactone and 1,4-dioxan-2-one.

The nonabsorbable component is in the form of a sheet. The bioabsorbable component is laminated to at least one side of the sheet. In a more specific embodiment, the bioabsorbable component is laminated to both sides of the sheet. The nonabsorbable component is in the form of a knitted, woven, or flat braided, sheet. In a preferred embodiment, the nonabsorbable component is in the form of a woven sheet.

In an alternative surgical composite structure for mammalian tissue
the nonabsorbable reinforcing component is prepared from a plurality of fibers.

In a specific embodiment, the bioabsorbable component is manufactured from the monomers glycolide and l,3-dioxan-2-one. In another specific embodiment, the nonabsorbable component is in the form of a knitted or woven sheet.

In another alternative surgical composite structure for mammalian tissue
the nonabsorbable woven component is prepared from a plurality of fibers.

Furthermore, the nonabsorbable woven component may also comprise members selected from the group consisting of polytetrafluoroethylene, a copolymer of tetrafluoroethylene and hexafluoropropylene, and polyvinylidene fluoride.

In yet another alternative surgical composite structure for mammalian tissue
the nonabsorbable woven component is prepared from a plurality of fibers, the fibers comprising a polybutester, and
the bioabsorbable component is laminated to the nonabsorbable woven component, the bioabsorbable component comprising a random copolymer prepared from at least the monomers glycolide and 1,3-dioxan-2-one.

A drawing which describes the shape and/or geometrical configuration of the surgical composite structure is not necessary for an understanding of this invention. That is, any person skilled in the surgical composite structure art will know how to manufacture and how to use the invention by reading this specification, generally and the examples, specifically.

It is to be understood that the bioabsorbable component can be coated onto the nonabsorbable reinforcing component by any coating means known in the prior art. The inventors have found that lamination is an adequate means for coating. Specifically, the inventors have found that bonding the bioabsorbable component to the nonabsorbable reinforcing component by fusion is an adequate means of laminating the two components. However, other forms of coating, such as encapsulation, are within the scope of this invention.

Throughout this disclosure, it is to be understood that the term Teflon is a trademark of the E. I. DuPont and Company, DE, U.S.A., whether the term Teflon is or is not so identified as a trademark.

The term fluoropolymer is generic and includes the terms fluoroplastic and fluoroelastomer.

The Teflon™ FEP described in the examples is a copolymer of tetrafluoroethylene and hexafluoropropylene. It is also to be understood that the term polybutester as used in this disclosure is synonymous with the terms polyetherester, polyether-ester or polyether ester. A commercially available polybutester is the Hytrel™ (E. I. DuPont and Co.) copolymer.

A preferred embodiments of this invention is more fully described in the Example, below.

Reference Examples 1-3 are only incorporated for illustrative purposes and are outside the scope of the claim.

### REFERENCE EXAMPLE 1

A woven mesh, identified as Style T-151-56 and supplied by Stern & Stern Textiles, Inc., Hugnet Fabrics Div., Hornell, NY 14843, U.S.A. is used as a control for the composite meshes of Examples 1 and 2, below. It is made with monofilament fibers of Teflon™ FEP (E. I. DuPont and Co.) polymer with a diameter of 0.013-0.015 cm (or 0.005 inch to 0.006 inch). These fibers are woven into a mesh configuration of approximately 80 x 90 strands per inch. The overall thickness of the woven fabric is approximately 12 mils (or 0.012 inch).

### REFERENCE EXAMPLE 2

A composite structure consisting of a fabric of a woven mesh made up of biocompatible, nonabsorbable, monofilament fibers is encapsulated by lamination between two films of a bioabsorbable polymer. The woven mesh is described in Reference Example 1, above.

The films of bioabsorbable polymer used in the laminated composite are prepared by compression molding a random copolymer of 50/50 weight per cent of glycolide trimethylene carbonate to a thickness of approximately 0.025 cm (or 0.010 inch) thick. The manufacture of the random copolymer is described, without the need for undue experimentation, in the prior art.

The conditions for molding these films is as follows. A pre-dried, preformed pellet of approximately 5.0 grams of the copolymer is placed between two 17.8 x 17.8 cm (7 inch x 7 inch) caul plates and Teflon PTFE release film. This assembly is placed in a (6 inch x 6 inch) 15.2 x 15.2 cm press which is pre-heated to 145°C ± 5°C and gradually increased in pressure to 454 kg (1,000 lbs.) force on a 4.4 cm (1 3/4 inch) diameter ram. The pressure is held constant for 3 minutes. Subsequently, this assembly is removed from the press, and cooled to approximately 15°C under pressure. The film is then easily released from between the Teflon release film. The resultant film is a transparent, amber-colored film of approximately 0.025 cm thick x 12.7 cm x 12.7 cm ((0.010 inch) thick x 5 inches x 5 inches).

The final composite structure is formed by laminating a film of the copolymer on either side of the Teflon FEP woven mesh between two 17.8 x 17.8 cm (7 inch x 7 inch) caul plates and Teflon PTFE release film. This assembly is placed in a 15.2 x 15.2 cm (6 inch x 6 inch) press which is pre-heated to 145°C ± 5°C and gradually increased in pressure to 454 kg (1,000 lbs.) force. The pressure is held constant for approximately 3 minutes. Subsequently, this assembly is removed from the press and cooled to approximately 15°C under pressure. The laminated composite structure is then easily released from between the Teflon PTFE release film. the resultant laminated composite consists of a Teflon FEP woven fabric which is completely encapsulated within the fused copolymer of 50/50 weight percent poly(glycolide-co-trimethylene carbonate). The resultant laminated composite is approximately 0.051 cm thick and 12.7 x 12.7 cm ((0.020 inch) thick and 5 inches x 5 inches) in size.

### REFERENCE EXAMPLE 3

A composite laminated structure is prepared similar to that described in Reference Example 2 except the polymer films are made with a random copolymer of 68/32 weight per cent of glycolide trimethylene carbonate substituted for the 50/50 weight per cent copolymer. Also, the processing temperature was increased to approximately 150°C ± 5°C.

The preparation of the random copolymer is described, without the need for undue experimentation, in the prior art.

### EXAMPLE

A composite laminated structure is prepared similar to that described in Reference Example 3 except that the woven mesh is made from monofilament fibers of HYTREL® with a diameter of 0.013 cm to 0.015 cm (0.005 inch to 0.006 inch). Hytrel™ (E. I. DuPont and Co.) is a polyether-ester, and can be a polymer of polytetramethylene glycol with terephthalic acid and 1,4 - butanediol. These fibers are woven into a mesh configuration of approximately 80 x 90 strands per 2.54 cm (1 inch). The overall thickness of the woven fabric is approximately 0.03 cm (0.012 inch).

## Claims

1. A surgical composite structure for mammalian tissue comprising:
a) a nonabsorbable reinforcing component prepared from one or more fibres, at least one of the fibres manufactured from a polymer selected from the group consisting of a fiber forming polyether-ester and blends of the same, and
b) a synthetic bioabsorbable component comprising a random copolymer prepared from at least the monomers glycolide and 1,3-dioxane-2-one
wherein the nonabsorbable component (a) is in the form of a knitted, woven, or flat braided sheet and the bioabsorbable component (b) is laminated to at least one side of the sheet.

## Patentansprüche

1. Chirurgische Verbundstruktur für Säugergewebe, umfassend:
a) eine nichtabsorbierbare, verstärkende Komponente, hergestellt aus einer oder mehreren Fasern, wobei mindestens eine der Fasern aus einem Polymer, ausgewählt aus der Gruppe, bestehend aus faserbildendem Polyetherester und Gemischen davon hergestellt ist, und
b) eine synthetische, bioabsorbierbare Komponente, umfassend ein statistisches Copolymer, hergestellt aus mindestens den Monomeren Glycolid und 1,3-Dioxan-2-on,
wobei die nichtabsorbierbare Komponente (a) in Form einer geknüpften, gewebten oder flach geflochtenen Lage vorliegt und die bioabsorbierbare Komponente (b) auf zumindest eine Seite der Lage laminiert ist.

## Revendications

1. Structure composite chirurgicale pour tissus de mammifères comprenant:
a) un constituant de renfort non absorbable préparé à partir d'une ou de plusieurs fibres, au moins l'une des fibres étant fabriquée à partir d'un polymère choisi dans le groupe constitué par un polyétherester formant une fibre et des mélanges de polyétheresters, et
b) un constituant synthétique bioabsorbable comprenant un copolymère statistique préparé au moins à partir des monomères glycolide et 1,3-dioxane-2-one
dans laquelle le constituant non absorbable (a) est sous forme d'une feuille tricotée, tissée ou tressée à plat, et le constituant bioabsorbable (b) est stratifié sur au moins une face de la feuille.
